# EUROPEAN PATENT SPECIFICATION

(11) **EP 3 727 546 B1**
(45) Date of publication and mention of the grant of the patent: **15.01.2025**
(21) Application number: 18833649.9
(22) Date of filing: 20.12.2018
(51) Int. Cl.: A61M 16/08, B29C 48/00

(54) **RESPIRATORY TUBE AND METHOD OF PRODUCING THEREOF**
BEATMUNGSTUBUS UND VERFAHREN ZUR HERSTELLUNG DAVON
TUBE RESPIRATOIRE ET SON PROCÉDÉ DE PRODUCTION

(30) Priority: 22.12.2017 GB 201721763
(43) Date of publication of application: 28.10.2020
(73) Proprietor: Intersurgical AG, 9490 Vaduz (LI)
(72) Inventor: MILLER, Andrew Neil, Wokingham, Berkshire RG41 2RZ (GB)
(74) Representative: Adamson Jones
(86) International application number: PCT/EP2018/086204
(87) International publication number: WO 2019/122133

(56) References cited:
- WO-A1-01/13021
- WO-A1-01/34366
- GB-A- 2 268 786
- GB-B- 2 496 569

## Description

The present disclosure relates to medical tubing, particularly to medical tubing designed to carry a liquid or gas at elevated or lowered temperature.

Tubing is used in a wide range of medical applications, for the transfer of fluids and/or gases to and from a patient or device. In many cases, the liquid or gas being transferred is at an elevated or lowered temperature, compared to ambient air. In such applications, both maintenance of the elevated or lowered temperature and the ability to observe the interior of the tube for condensation are important.

This can be particularly important in ventilation tubes for use in respiratory circuits.

In a healthy person, breathing is entirely spontaneous. The brain senses a build-up of carbon dioxide in the blood and immediately calls for more oxygen. This oxygen is taken into the body by spontaneous inspiration and carbon dioxide is removed in the passive exhalation phase of respiration.

The upper airway of a healthy person functions to filter, warm and humidify inspired air, and in turn to capture heat and moisture during expiration. It is essential that the humidity within the upper airway is maintained at a sufficiently high level to ensure efficient gas exchange and, if necessary, to maintain the mucociliary transport system. The mucociliary transport system is responsible for trapping inhaled contaminants and removing them from the lung. Compromised mucociliary transport can result in reduced airway patency and lung compliance, and can increase the risk of infection.

When a patient is ventilated using a respiratory circuit, the upper airway is often bypassed using an endotracheal tube, further reducing the ability to humidify inspired air. However, a sufficient humidity can be achieved using conventional apparatus, such as a heat-moisture exchanger (HME) or a heated water bath humidifier. In the case of a heated water bath, as humid inspiratory gas travels along the respiratory circuit, a certain amount of water vapour will cool and start to condense, forming water droplets, which will start to build up, causing so-called "rain-out". A quantity of rain-out indicates a reduction in humidity delivery to the patient and excess rain-out can cause occlusion of the ventilation tube, and potentially damage the ventilator or anaesthetic equipment. Damaged equipment, for example a damaged valve or flow sensor, can lead to incorrect ventilation of the patient.

This water may occlude the respiratory air flow or drain back into the patient's lungs thereby putting the patient at risk of drowning, and may also drain into the ventilator/anaesthetic equipment thus causing damage. If water is allowed to accumulate over a prolonged period then due to its non-compressible nature the water will effectively block the respiratory circuit.

Both prevention of and monitoring of condensed water within a respiratory circuit are required to reduce the risks of an adverse outcome. Typically, condensation is prevented through the use either of a heated, or an insulated tube. Tube insulation has been achieved through the use of tubes having walls formed of a foamed material, which acts as an insulator due to the air trapped within the structure of the foam. However, foamed materials are typically partially or completely opaque, and it is therefore difficult to monitor for the formation of condensate within the tube during use.

An example of a breathing tube comprising a foamed wall is described in GB2496569. Visualisation of the inside of the tube is achieved by making the wall of the tube of a sufficient minimum optical transparency that it is possible to visually detect liquid through the wall of the tube. This may be achieved, for example, by using a thinner wall, or by reducing the degree to which the wall is foamed, either of which will reduce the insulating benefits of using a foamed wall.

The document GB2268786A describes another prior art respiratory tube comprising a corrugated tubular body.

There has now been devised an improved respiratory tube and method of manufacturing a respiratory tube that overcomes or substantially mitigates the above mentioned and/or other problems associated with the art.

### Summary of the invention

The present invention provides a respiratory tube according to claim 1, and a method for producing a respiratory tube according to claim 13. Further preferred embodiments of the invention are defined in the dependent claims.

### Summary of the present disclosure

Reference will now be made to various exemplary embodiments or aspects of the present disclosure. Embodiments or aspects disclosed herein which do not fall under the scope of the appended claims do not form part of the present invention, but are useful for understanding the principles of the invention. The scope of the present invention is defined by the appended claims.

According to a first aspect of the present disclosure there is provided a respiratory tube comprising a tubular body, the walls of the tubular body comprising a foamed material and a non-foamed material, wherein at least part of the non-foamed material forms at least one transparent portion.

The use of a foamed material in the walls of the respiratory tube provides insulation, helping to prevent condensation from forming when humidified gas passes through the tube. Simultaneously, the transparent portion(s) enable the interior of the tube to be easily monitored for the formation of condensation. As the interior of the tube is visualised through the non-foamed transparent portion(s), rather than through the foamed material, a more insulating foam (one which contains more gas bubbles) may be used than would otherwise be possible. Thus it has been found that, by combining foamed and transparent portions, sufficient insulation may be achieved whilst at the same time allowing easy viewing of the interior of the tube.

The at least one transparent portion may extend longitudinally along the tubular body. "Longitudinally" means, in the context of the present disclosure, a feature which extends along the length of the tube, and encompasses features which are straight, curved and/or helical. Thus, the at least one transparent portion may extend in a straight line down the tubular body, or may wind helically about the tubular body. In the respiratory tube according to the present invention, the at least one transparent portion extends longitudinally along the tubular body.

The at least one transparent portion may alternatively take the form of an individual window, or of a band of transparent material about the circumference of the tube.

It may be particularly advantageous for the respiratory tubing to comprise from 3 to 5 transparent portions, e.g. 3 transparent portions. Multiple transparent portions spaced about the circumference of the tube enables the interior of the tube to be viewed from multiple angles, so that the interior of the tube can be viewed regardless of the relative angle of the viewer. The benefit of being able to view the interior of the tube from multiple angles must be balanced against the need to retain the insulating properties of the tube, by maintaining a sufficient proportion of foamed material.

By "transparent" is meant, in the context of the present disclosure, a material through which it is possible to see whether water is present. Thus, a "transparent" portion is one through which it is possible to visualise condensate in the interior of the tube. "Transparent" may therefore encompass semi-transparent and translucent materials.

The non-foamed material in the respiratory tubing of the first aspect of the present disclosure is transparent, and may be any suitable transparent material known in the art. The non-foamed material may be a polymeric material, for example polyethylene, ethylene vinyl acetate (EVA), polyurethane, silicone rubber or Hytrel^{®}, or a synthetic rubber such as neoprene, nitrile rubber or EPDM.

The combination of a foamed and a non-foamed material in a respiratory tube also has reinforcing benefits when the non-foamed material extends longitudinally along the tubular body. The longitudinal non-foamed material acts to strengthen the tube when compared to a tube formed entirely from foamed material. This forms a tube that is self-supporting without the need to add an additional strengthening rib, reducing manufacturing costs.

Thus, according to a second aspect of the present disclosure there is provided a respiratory tube comprising a tubular body, the walls of the tubular body comprising a foamed material and a non-foamed material, wherein at least part of the non-foamed material forms at least one reinforcing portion that extends longitudinally along the tubular body.

The at least one reinforcing portion may be transparent.

The non-foamed material in the respiratory tubing of the second aspect of the present disclosure may be any suitable material known in the art. The non-foamed material may be a polymeric material, for example polyethylene, ethylene vinyl acetate (EVA) or polyurethane.

The structural and functional features described below are described in connection with both the first and second aspects of the present disclosure.

The dimensions of tubes suitable for use in conjunction with respiratory equipment are known in the art, and tubes of the present invention may have any suitable dimensions to enable them to be used with conventional equipment. For example, the respiratory tubes of the invention may have an internal diameter of from 5mm to 35mm, and may more particularly have an internal diameter of 6mm, 10mm, 13mm, 15mm, 19mm, 22mm, 25mm or 32mm.

In addition, the respiratory tube may further comprise connectors to enable it to be coupled to existing ventilation equipment. The respiratory tube may have a connector at each end.

The thickness of the wall of the respiratory tube may be invariable, or may vary about its circumference.

The tubular body may be corrugated. Corrugated tubing is more flexible, and has a lower likelihood of being crushed or kinked than non-corrugated tubing. The respiratory tube according to the present invention comprises a corrugated tubular body.

The foamed material in the respiratory tubing may form at least one foamed portion. The foamed portion(s) may be non-transparent.

By "non-transparent" is meant, in the context of the present disclosure, that it is not possible to view an image through the non-transparent material. Thus, a "non-transparent" material is one through which it is not possible to visualise condensate on the interior of the tube. The "non-transparent" material may be opaque, or it may be possible to see a diffuse light source which is located behind the material. Thus, it is not possible to visualise condensate in the interior of the tube through the foamed portion(s), however, it may be possible to see a diffuse light source which is located behind the foamed material.

The foamed material may be an open celled foam or a closed celled foam. The foamed material may be resilient, such that it returns to its original form after flexing or compression. The foamed material may be any suitable foam, for example a foamed plastics material, for example a foamed polyethylene, ethylene vinyl acetate (EVA), neoprene, polyurethane, polyvinyl chloride (PVC), Hytrel^{®}, or a blend, such as a polyolefin or ethylene polymer blend.

The foamed portion(s) may extend longitudinally down the length of the tube. The foamed portion(s) may extend in a line parallel to the transparent or reinforcing portion(s).

The foamed material may have a density of between 20% and 80% of the density of the material prior to foaming.

The tubing may comprise a plurality of foamed portions. For example, the respiratory tubing may comprise at least two, or at least three, or at least four foamed portions. The plurality of foamed portions may be equally spaced about the circumference of the tube.

Where there are a plurality of foamed portions, the foamed portions may be entirely separated from each other by the non-foamed material.

In all embodiments, the foamed material may account for anything up to, but not including, 100% by volume of the walls of the respiratory tubing. For example, the non-foamed material may account for between 0.5% and 99.5% by volume of the walls of the respiratory tubing, or between 3% and 97%, or between 10% and 90% by volume of the walls of the respiratory tubing.

The foamed material may cover at least 50% of the surface area of the outside of the tube, or at least 60%, at least 70%, at least 80%, at least 90%, or at least 95% of the surface area of the outside of the tube. The foamed material may cover up to 99% of the surface area of the outside of the tube.

In one embodiment, the non-foamed material may extend about the whole circumference of the tube, overlaid or underlaid at intervals by the foamed material. Thus, strips of foamed material may overlay a tube formed of a non-foamed material. The strips of foamed material may be spaced at intervals about the circumference and extend longitudinally along the length of the tube, such that areas of non-foamed material are exposed between the strips of foamed material.

On the outer surface of the tube, the surface of the foamed material may be flush with the surface of the non-foamed material. Alternatively, the surface of the foamed material may be higher or lower than the surface of the non-foamed material, creating a step or ridge between the foamed material and the non-foamed material. In this embodiment, the cross-sectional shape of the inner wall of the tube may mirror the shape of the outer wall, eg circular, or may be an irregular shape. For example, the inner wall of the tube may be ridged, with the ridges corresponding to the locations where the foam material has been overlaid on the exterior of the tube. In all embodiments, the non-foamed material may account for anything up to, but not including, 100% by volume of the walls of the respiratory tubing. For example, the non-foamed material may account for between 0.5% and 99.5% by volume of the walls of the respiratory tubing, or between 3% and 97%, or between 10% and 90% by volume of the walls of the respiratory tubing.

The non-foamed material may cover less than 50% of the surface area of the outside of the tube, or less than 40%, less than 30%, less than 20%, less than 10% or less than 5% of the surface area of the outside of the tube. The non-foamed material may cover at least 1% of the surface area of the outside of the tube.

The respiratory tube may comprise a plurality of transparent or reinforcing portions.

Each of the plurality of transparent or reinforcing portions may extend longitudinally along the tubular body, spaced about the circumference of the tube. For example, the respiratory tube may comprise at least two, or three, or four, or five transparent or reinforcing portions. The plurality of transparent or reinforcing portions may be spaced at substantially regular intervals about the circumference of the tube, separated by non-transparent portions comprising a foamed material.

The transparent or reinforcing portion(s) may be relatively narrow when compared to the width of the foamed portions, to maintain the benefit of the insulating properties of the foamed material. However, the transparent portion(s) must be of sufficient width that condensate on the interior of the tube can be seen. The width of the transparent or reinforcing portions may be from about 1mm to about 5mm, or from about 2mm to about 4mm, or from about 2mm to about 3mm.

The comparative width of the foamed and transparent or reinforcing portions is in part responsible for determining the degree of insulation of the tube. Thus, the width of the transparent or reinforcing portion(s) may be less than 50% of the width of the foamed portion(s), or less than 40% of the width of the foamed portion(s), less than 30% of the width of the foamed portion(s) or less than 20% of the width of the foamed portion(s).

Typically, the foamed material overlays or underlays the non-foamed material, but is not encapsulated by it. Thus, the foamed material forms at least part of the outer and/or the inner surface of the tubular body. This enables the tubular body to be formed either by co-extruding the foamed and non-foamed materials, or to be formed by just two extrusion dies set closely together.

In one embodiment, two or more transparent or reinforcing portions may be connected via a bridge of non-foamed material extending above, through or below the foamed material. For example, the non-foamed material may extend about the circumference of the tube, overlaid or underlaid at intervals by the foamed material. Thus, the entire inner circumference of the tube may be formed of the non-foamed material. Longitudinal strips of foamed material may extend along the tubular body, partially overlaying the tube of non-foamed material. Exposed region(s) of non-foamed material between the strip(s) of foamed material form the at least one transparent or reinforcing portion(s).

A plurality of strips, e.g. at least 2, 3, 4 or 5 strips, of foamed material may be located at intervals about the circumference of the tubular body.

In this embodiment, the surface of the foamed material may be flush with the surface of the non-foamed material, or may be raised or lowered in comparison to it. The tube wall may be of consistent thickness, or may vary in thickness about its circumference.

In an alternative embodiment, the wall of the tubing may be formed of alternating longitudinal strips of foamed and non-foamed material, with the strips of foamed material entirely separating the strips of non-foamed material. In the first aspect of the present disclosure, the strips of non-foamed material form transparent windows through which the interior of the tube can be viewed, while the foamed material insulates the tube.

The strips of foamed and non-foamed material may be of equal depth, such that the thickness of the tube wall is consistent about its circumference.

Alternatively, the strips of foamed material and non-foamed material may be of different depth, such that the thickness of the tube wall varies about its circumference. The portions of the wall formed by the non-foamed material may have a thickness which is less than the thickness of the portions of the wall formed by the foamed material. The reduction in the thickness of the non-foamed material lightens the tube, while the thicker foamed portions provide greater insulation. Thus, it is possible to produce a tube having greater insulation due to the thickness of foam which can be incorporated, without compromising on the ability to visualise the interior of the tube or increasing the weight of the tube. There may be a step change in thickness between the foamed and transparent or reinforcing portions on the exterior of the tube, on the interior of the tube, or both.

The foamed portion(s) may extend longitudinally down the length of the tube.

The foamed and transparent or reinforcing portion(s) may extend parallel to each other down the length of the tube.

The foamed and transparent or reinforcing portion(s) may extend down the length of the tube in substantially straight lines.

The foamed and transparent or reinforcing portion(s) may extend helically along the length of the tube.

As the foamed portion(s) extend longitudinally down the length of the tube, the foamed portion(s) may be unbroken. That is, the foamed portion(s) may extend in an unbroken line along the length of the respiratory tube.

As the transparent or reinforcing portion(s) extend longitudinally down the length of the tube, the transparent or reinforcing portion(s) may be unbroken. That is, the transparent or reinforcing portion(s) may extend in an unbroken line along the length of the respiratory tube.

Either of the foamed or non-foamed materials may be breathable. Breathable medical tubing allows excess moisture to pass through the tube and evaporate into the atmosphere, reducing the build-up of excess condensation within the tube.

Thus, the respiratory tube of either the first or second aspect of the present disclosure may be breathable.

Either the foamed material, the non-foamed material, or both, may be breathable.

By "breathable" is meant, in the context of the present disclosure, that the material is permeable to water vapour and/or liquid water.

Suitable breathable foamed materials are typically open-celled, or partially open-celled foams, containing voids through which gases or fluids can pass.

Breathable non-foamed materials are known in the art, and any suitable material may be used. The breathable non-foamed material may be a polymeric material. Suitable breathable non-foamed materials include Hytrel^{®}, Nation^{®} and polyurethane.

The respiratory tubing of the first and second aspects of the present disclosure may particularly be used in conjunction with therapies in which humidification of the ventilated air is desirable or required. For example, the respiratory tubing may be used in intensive care, or in long-term therapies, where it is necessary to bypass the upper airway of the patient (tracheostomy). However, the tubing may also find utility in any situation in which it is necessary to transport gases to and/or from a patient.

In a further aspect of the present disclosure there is provided a method for producing a respiratory tube comprising a corrugated tubular body, the method comprising extruding a tubular body and, following extrusion, shaping the tubular body using a corrugator die chain, the walls of the corrugated tubular body comprising a foamed material and a non-foamed material, wherein at least part of the non-foamed material forms at least one transparent portion extending longitudinally along the corrugated body, and wherein the interior of the tube is visible through the at least one transparent portion.

The foamed material and the non-foamed portions of the respiratory tube may be formed simultaneously.

The foamed and non-foamed material may be co-extruded to form the respiratory tube. For example, adjacent strips of foamed and non-foamed material may be co-extruded to produce a respiratory tube having alternating strips of foamed and non-foamed material about its circumference. This ensures adherence of the two materials, maintaining the integrity of the tube. Following extrusion, the tube is shaped by a corrugator die chain where a corrugated tube is desirable.

There is thus provided a method for manufacturing a tube according to the first or second aspect of the present disclosure comprising co-extruding lines of a foamed material and a non-foamed material through a die head to produce a tube having alternating stripes of foamed and non-foamed material.

In embodiments in which the at least one transparent portion extends helically along the tubular body, the helical structure may be formed by co-extruding the non-foamed and foamed materials using a rotating die head.

Alternatively, the foamed material and the non-foamed material may be formed separately, or sequentially. The non-foamed material may first be extruded into a tube, with strips of the foamed material subsequently being applied on top of the non-foamed material, extending longitudinally along the tube.

Where the foamed material is extruded onto the surface of the non-foamed material almost immediately, the foamed material may sink into the non-foamed material, forming a flush or almost flush surface.

There is thus provided a method for manufacturing a tube according to the first aspect of the present disclosure comprising the following steps:
a) extruding a tube of non-foamed material;
b) extruding stripes of foamed material on the tube of step a), such that portions of the non-foamed material are left exposed between the stripes.

In embodiments in which the at least one transparent or reinforcing portion extends helically along the tubular body, the helical structure may be formed by extruding the non-foamed material using a rotating die head.

The tube is subsequently be passed through a corrugator die chain, to produce a corrugated tube.

The respiratory tube may be used in conjunction with a breathing circuit, and may be used as inspiratory and/or expiratory tubing. The respiratory tube may, in use, be connected to breathing apparatus, transporting inhaled and/or exhaled air between eg a ventilator and a patient.

The respiratory tubing may further comprise a connector at each end, to permit the tube to be connected to the required apparatus.

In a further aspect of the present disclosure there is thus provided a breathing circuit comprising a patient interface and a ventilator, the patient interface and ventilator being fluidly connected by a respiratory tube according to the first aspect of the disclosure.

The breathing circuit may be an open circuit, in which the exhaled air is vented to the atmosphere. In this case, the respiratory tube may be used as an inspiratory tube to transport gas from the ventilator to the patient, for inhalation.

The breathing circuit may be a closed circuit, in which the exhaled air is returned to the ventilator. The respiratory tube may be used as either the inspiratory tube, or the expiratory tube, or both.

The respiratory tube may be provided with a connector at each end to permit attachment to the patient interface and ventilator.

The patient interface may be any interface by which gases are introduced to a patient. Examples include, but are not limited to a tracheostomy tube, endotracheal tube, cannula, full face mask, nasal mask, an NIV mask, or masks designed for CPAP or BPAP. A respiratory tube according to the present disclosure is of particular utility in situations where it is necessary for the air to be humidified, for example in intensive care or other long-term therapies in which the upper airway of the patient is bypassed.

Embodiments of the present disclosure will now be described, by way of example only, by reference to the accompanying Figures.
Figure 1 is a perspective view of a respiratory tube according to the disclosure;
Figure 2 is a cross-sectional view of a respiratory tube according to a first embodiment of the invention;
Figure 3 is a cross-sectional view of a respiratory tube according to a second embodiment of the invention;
Figure 4 is a cross-sectional view of a respiratory tube according to a third embodiment of the invention;
Figure 5 is a cross-sectional view of a respiratory tube according to a fourth embodiment of the invention;
Figure 6 is a schematic diagram showing the method of manufacture of a respiratory tube of the invention.

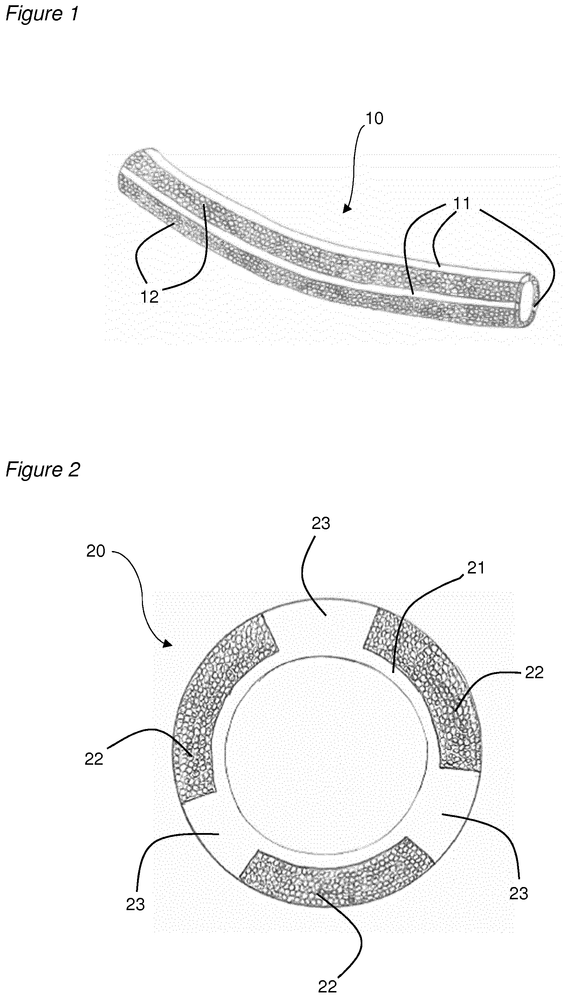

Figure 1 shows a respiratory tube 10 having transparent portions 11. The transparent portions 11 are formed of a non-foamed material, eg polyethylene, and extend longitudinally along the length of the tube. The respiratory tube 10 has three transparent portions 11 evenly spaced about the circumference of the tube, enabling the interior of the tube to be viewed from multiple directions. It will be appreciated that more or less transparent portions could be used, for example 2, or 4, or 5 transparent portions spaced about the circumference of the tube.

Foamed portions 12 are located between the transparent portions 11, and extend longitudinally along the length of the tube, parallel to the transparent portions 11. The foamed portions 12 may be formed of foamed polyethylene or of any other suitable material (eg ethylene vinyl acetate (EVA), neoprene, polyurethane, polyvinyl chloride (PVC), Hytrel^{®}, or a blend, such as a polyolefin or ethylene polymer blend), and are of sufficient opacity that the interior of the tube cannot be viewed through the foamed portions 12.

The width of each transparent portion 11 is substantially smaller than the width of each foamed portion 12, being approximately one third the width. Thus, the foamed material accounts for approximately 75% of the surface area of the tube 10. The foamed portions 12 and transparent portions 11 alternate around the circumference of the tube, each transparent portion 11 being separated from the remaining transparent portions by a foamed portion 12. The wall thickness is consistent around the circumference of the tube: the transparent portions 11 and the foamed portions 12 have the same depth.

The respiratory tube 10 may also be corrugated.

Figure 2 depicts a cross-sectional view of a respiratory tube 20. The respiratory tube 20 is formed of a non-foamed material and a foamed material, eg polyethylene and foamed polyethylene. A wall 21 of non-foamed material extends about the entire inner circumference of the tube. On the outer surface of the wall 21, three ridges of non-foamed material, which form the transparent portions 23, are spaced about the circumference of the tube and extend longitudinally along the length of the tube. Foamed material 22 fills the spaces between the transparent portions 23. The foamed material 22 extends longitudinally along the length of the tube, in the manner shown in Figure 1. Where the foamed material 22 overlays the wall 21, it prevents that portion of the tube from being transparent and provides insulation.

Thus, transparent portions 23 formed of non-foamed material are disposed between the sections of foamed material 22, and may extend along the length of the tube in the manner depicted in Figure 1, or may wind helically about the tube. The transparent portions 23 may be formed of the same material, for example polyethylene, as the wall 21.

The width of the transparent portions 23 is approximately half that of the sections of foamed material 22. Thus, the foamed material accounts for approximately 66% of the surface area of the tube 20. On the outer wall of the tube, the sections of foamed material 22 are flush with the transparent portions 23. According to the present invention (but not shown in the figure), the tube is corrugated.

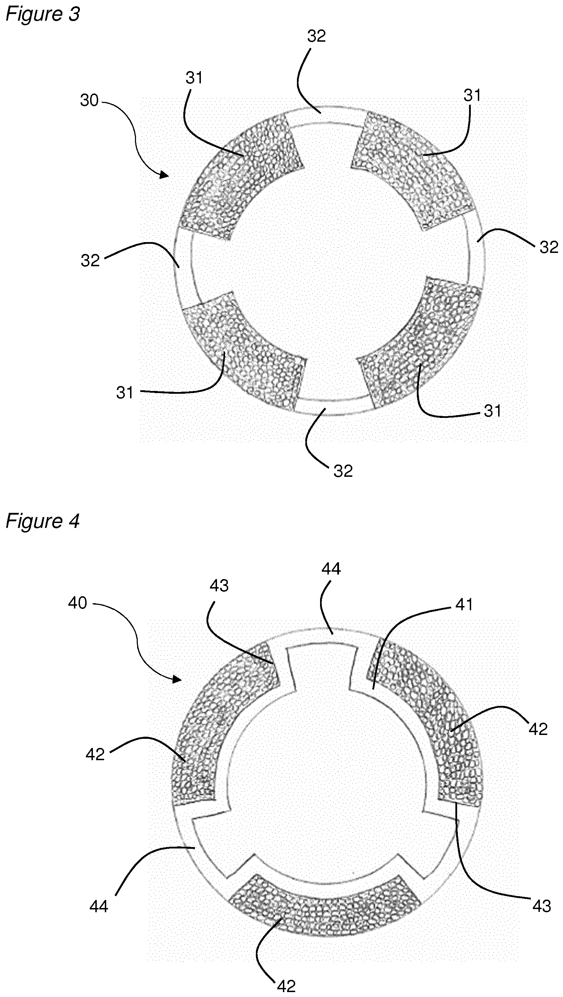

Figure 3 shows a cross-sectional view of an alternative embodiment of the invention.

The walls of respiratory tube 30 are formed of alternating foamed portions 31 and non-foamed portions 32, the non-foamed portions being transparent and allowing condensate on the interior of the tube to be seen.

The depth non-foamed portions 32 is less than the depth of the foamed portions 31 , reducing the weight of the tube whilst maintaining a high degree of insulation. The foamed portions 31 and non-foamed portions 32 are flush with each other about the outer wall of the tube, but are offset from each other about the inner wall of the tube.

According to the present invention (but not shown in the figure), the tube is corrugated.

Figure 4 depicts a further embodiment of the respiratory tube of the invention. The respiratory tube 40 is formed of a foamed material and a non-foamed material. The non-foamed material forms an irregularly shaped wall 41 , which extends about the entire inner circumference of the tube. The irregular shape of the wall 41 forms a series of troughs 43 and peaks. Foamed material 42 fills the troughs 43 at three equally spaced intervals around the circumference of the tube, overlaying the wall 41 at those points. The foamed material 42 may extends along the length of the tube, in the manner shown in Figure 1, or may wind helically about the tube. Where the foamed material 42 overlays the wall 41, it prevents that portion of the tube from being transparent and provides insulation.

Those portions of the wall 41 which remain exposed between the sections of foamed material 42 form transparent portions 44 through which condensate on the interior of the tube can be viewed. During manufacture, the non-foamed material is first extruded through a shaped die to form the irregularly shaped wall 41. The foamed material 42 is subsequently extruded into the troughs 43, forming foamed portions and leaving transparent portions 44 exposed. According to the present invention (but not shown in the figure), the tube is corrugated.

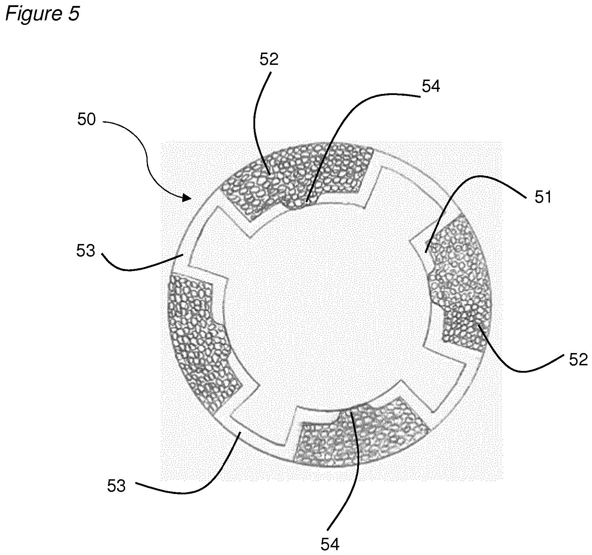

Figure 5 illustrates an embodiment of the invention similar to that of Figure 4, comprising a tube 50 having an irregularly shaped wall 51, foamed portions 52 and transparent portions 53. Figure 5 shows how the wall 51 of the tube may be distorted during manufacture, when the foamed material is applied. As the foamed material is applied before the non-foamed material has set, the weight of the foamed material may displace some of the non-foamed material. This displacement may be small or, as shown in Figure 5, may cause the wall 51 to be interrupted at a number of points 54 by the foamed material. This displacement may also be seen during manufacture of the embodiment of the respiratory tube shown in Figure 2. According to the present invention (but not shown in the figure), the tube is corrugated.

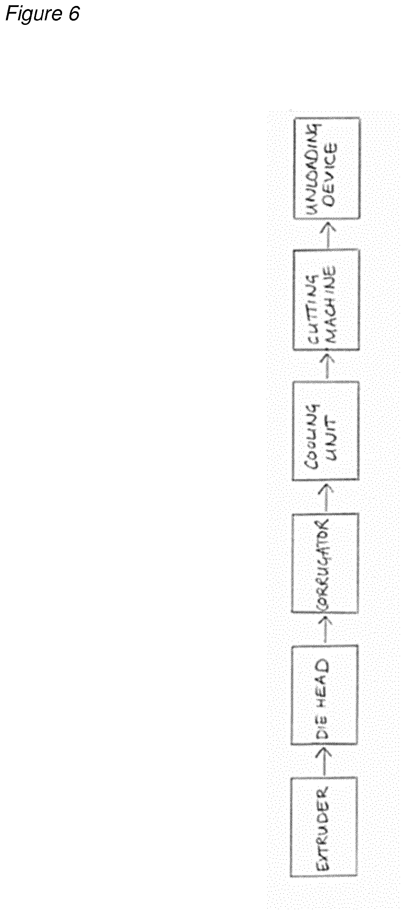

Figure 6 is a schematic diagram showing a method by which respiratory tubes of the invention may be manufactured. The foamed and non-foamed materials are extruded, either simultaneously or sequentially, through die heads, to form the basic tube. Rotating die heads are used to form a helical structure. The tube is then passed through a corrugator, before the final form of the tube is set by a cooling machine and cut to the required size.

In order to form respiratory tube 20 as shown in Figure 2, respiratory tube 40 as shown in Figure 4 and respiratory tube 50 as shown in Figure 5, the non-foamed section is extruded first, with the foamed material being applied on top, prior to corrugation.

The respiratory tube 30 shown in Figure 3 is formed by co-extrusion of the foamed and non-foamed material through a single die head to maintain integrity of the tube.

It will be appreciated that Figures 1-5 are stylized views of the respiratory tubing depicting the comparative positions and relative sizes of the foamed and non-foamed portions, but that in practise extrusion of the foamed and non-foamed materials may result in some movement of the materials relative to one another and/or a softening of the sharp edges and corners depicted.

## Claims

1. A respiratory tube (20, 30, 40, 50) comprising a corrugated tubular body, the walls of the corrugated tubular body comprising a foamed material (22, 31, 42, 52) and a non-foamed material (23, 32, 44, 53), wherein at least part of the non-foamed material (23, 32, 44, 53) forms at least one transparent portion, **characterised in that** the at least one transparent portion extends longitudinally along the corrugated tubular body, and that the interior of the tube is visible through the at least one transparent portion.

2. A respiratory tube according to Claim 1, wherein the foamed material forms at least one non-transparent portion.

3. A respiratory tube according to Claim 2, wherein the at least one transparent portion extends helically along the corrugated tubular body.

4. A respiratory tube according to Claim 1, wherein the foamed material forms at least one non-transparent portion, and wherein the at least one transparent portion and the at least one non-transparent portion extend parallel to each other longitudinally along the length of the tube.

5. A respiratory tube according to Claim 2, wherein the at least one non-transparent portion extends longitudinally along the corrugated tubular body.

6. A respiratory tube according to any of Claims 3 to 5, wherein the width of the at least one transparent portion is less than 50% the width of the at least one non-transparent portion.

7. A respiratory tube according to any preceding claim, wherein the inner wall of the tube is formed of non-foamed material, and the foamed material partially overlays the inner wall.

8. A respiratory tube according to any of Claims 1 to 6, wherein the wall of the respiratory tube is formed of alternating portions of foamed and non-foamed material extending longitudinally along the tubular body.

9. A respiratory tube according to Claim 8, wherein the portions of foamed material separate the portions non-foamed material.

10. A respiratory tube according to any preceding claim, wherein the surface of the foamed material is flush with the surface of the non-foamed material.

11. A respiratory tube according to any preceding claim, wherein the foamed material forms at least part of the outer surface of the corrugated tubular body.

12. A respiratory tube according to any preceding claim, wherein the tube is breathable.

13. A method for producing a respiratory tube (20, 30, 40, 50) comprising a corrugated tubular body, the method comprising extruding a tubular body and, following extrusion, shaping the tubular body using a corrugator die chain, the walls of the corrugated tubular body comprising a foamed material (22, 31, 42, 52) and a non-foamed material (23, 32, 44, 53), wherein at least part of the non-foamed material forms at least one transparent portion extending longitudinally along the corrugated body, and wherein the interior of the tube is visible through the at least one transparent portion.

14. A breathing circuit comprising a patient interface and a ventilator, the patient interface and ventilator being fluidly connected by a respiratory tube according to any of Claims 1 to 12.

## Patentansprüche

1. Beatmungsschlauch (20, 30, 40, 50), umfassend einen gewellten schlauchförmigen Körper, wobei die Wände des gewellten schlauchförmigen Körpers ein geschäumtes Material (22, 31, 42, 52) und ein nicht geschäumtes Material (23, 32, 44, 53) umfassen,
wobei mindestens ein Teil des nicht geschäumten Materials (23, 32, 44, 53) mindestens einen transparenten Abschnitt bildet, **dadurch gekennzeichnet, dass** sich der mindestens eine transparente Abschnitt in Längsrichtung entlang des gewellten schlauchförmigen Körpers erstreckt und dass das Innere des Schlauchs durch den mindestens einen transparenten Abschnitt sichtbar ist.

2. Beatmungsschlauch nach Anspruch 1, wobei das geschäumte Material mindestens einen nicht transparenten Abschnitt bildet.

3. Beatmungsschlauch nach Anspruch 2, wobei sich der mindestens eine transparente Abschnitt spiralförmig entlang des gewellten schlauchförmigen Körpers erstreckt.

4. Beatmungsschlauch nach Anspruch 1, wobei das geschäumte Material mindestens einen nicht transparenten Abschnitt bildet und wobei sich der mindestens eine transparente Abschnitt und der mindestens eine nicht transparente Abschnitt parallel zueinander in Längsrichtung entlang der Länge des Schlauchs erstrecken.

5. Beatmungsschlauch nach Anspruch 2, wobei sich der mindestens eine nicht transparente Abschnitt in Längsrichtung entlang des gewellten schlauchförmigen Körpers erstreckt.

6. Beatmungsschlauch nach einem der Ansprüche 3 bis 5, wobei die Breite des mindestens einen transparenten Abschnitts weniger als 50 % der Breite des mindestens einen nicht transparenten Abschnitts beträgt.

7. Beatmungsschlauch nach einem vorhergehenden Anspruch, wobei die Innenwand des Schlauchs aus nicht geschäumtem Material ausgebildet ist und das geschäumte Material die Innenwand teilweise überdeckt.

8. Beatmungsschlauch nach einem der Ansprüche 1 bis 6, wobei die Wand des Beatmungsschlauchs aus abwechselnden Abschnitten aus geschäumtem und nicht geschäumtem Material ausgebildet ist, die sich in Längsrichtung entlang des schlauchförmigen Körpers erstrecken.

9. Beatmungsschlauch nach Anspruch 8, wobei die Abschnitte aus geschäumtem Material die Abschnitte aus nicht geschäumtem Material trennen.

10. Beatmungsschlauch nach einem vorhergehenden Anspruch, wobei die Oberfläche des geschäumten Materials bündig mit der Oberfläche des nicht geschäumten Materials ist.

11. Beatmungsschlauch nach einem vorhergehenden Anspruch, wobei das geschäumte Material mindestens einen Teil der äußeren Oberfläche des gewellten schlauchförmigen Körpers bildet.

12. Beatmungsschlauch nach einem vorhergehenden Anspruch, wobei der Schlauch atmungsaktiv ist.

13. Verfahren zum Herstellen eines Beatmungsschlauchs (20, 30, 40, 50), der einen gewellten schlauchförmigen Körper umfasst, wobei das Verfahren Extrudieren eines schlauchförmigen Körpers und, nach dem Extrudieren, Formen des schlauchförmigen Körpers unter Verwendung einer Wellungsmatrizenkette umfasst, wobei die Wände des gewellten schlauchförmigen Körpers ein geschäumtes Material (22, 31, 42, 52) und ein nicht geschäumtes Material (23, 32, 44, 53) umfassen,
wobei mindestens ein Teil des nicht geschäumten Materials mindestens einen transparenten Abschnitt bildet, der sich in Längsrichtung entlang des gewellten Körpers erstreckt, und wobei das Innere des Schlauchs durch den mindestens einen transparenten Abschnitt sichtbar ist.

14. Beatmungskreislauf, einen Patientenanschluss und ein Beatmungsgerät umfassend, wobei der Patientenanschluss und das Beatmungsgerät durch einen Beatmungsschlauch nach einem der Ansprüche 1 bis 12 in Fluidverbindung stehen.

## Revendications

1. Tube respiratoire (20, 30, 40, 50) comprenant un corps tubulaire ondulé, les parois du corps tubulaire ondulé comprenant un matériau expansé (22, 31, 42, 52) et un matériau non expansé (23, 32, 44, 53),
au moins une partie du matériau non expansé (23, 32, 44, 53) formant au moins une partie transparente, **caractérisé en ce que** ladite au moins une partie transparente s'étend longitudinalement le long du corps tubulaire ondulé, et **en ce que** l'intérieur du tube est visible à travers ladite au moins une partie transparente.

2. Tube respiratoire selon la revendication 1, ledit matériau expansé formant au moins une partie non transparente.

3. Tube respiratoire selon la revendication 2, ladite au moins une partie transparente s'étendant de manière hélicoïdale le long du corps tubulaire ondulé.

4. Tube respiratoire selon la revendication 1, ledit matériau expansé formant au moins une partie non transparente, et ladite au moins une partie transparente et ladite au moins une partie non transparente s'étendant parallèlement l'une à l'autre longitudinalement le long de la longueur du tube.

5. Tube respiratoire selon la revendication 2, ladite au moins une partie non transparente s'étendant longitudinalement le long du corps tubulaire ondulé.

6. Tube respiratoire selon l'une quelconque des revendications 3 à 5, ladite largeur de ladite au moins une partie transparente étant inférieure à 50 % de la largeur de ladite au moins une partie non transparente.

7. Tube respiratoire selon l'une quelconque des revendications précédentes, ladite paroi interne du tube étant formée d'un matériau non expansé, et ledit matériau expansé recouvrant partiellement la paroi interne.

8. Tube respiratoire selon l'une quelconque des revendications 1 à 6, ladite paroi du tube respiratoire étant formée de parties alternées de matériau expansé et non expansé s'étendant longitudinalement le long du corps tubulaire.

9. Tube respiratoire selon la revendication 8, lesdites parties de matériau expansé séparant les parties de matériau non expansé.

10. Tube respiratoire selon l'une quelconque des revendications précédentes, ladite surface du matériau expansé affleurant la surface du matériau non expansé.

11. Tube respiratoire selon l'une quelconque des revendications précédentes, ledit matériau expansé formant au moins une partie de la surface externe du corps tubulaire ondulé.

12. Tube respiratoire selon l'une quelconque des revendications précédentes, ledit tube étant perméable à l'air.

13. Procédé de production d'un tube respiratoire (20, 30, 40, 50) comprenant un corps tubulaire ondulé, le procédé comprenant l'extrusion d'un corps tubulaire et, après extrusion, la mise en forme du corps tubulaire à l'aide d'une chaîne de filières onduleuses, les parois du corps tubulaire ondulé comprenant un matériau expansé (22, 31, 42, 52) et un matériau non expansé (23, 32, 44, 53),
au moins une partie du matériau non expansé formant au moins une partie transparente s'étendant longitudinalement le long du corps ondulé, et
ledit intérieur du tube étant visible à travers ladite au moins une partie transparente.

14. Circuit respiratoire comprenant une interface patient et un ventilateur, l'interface patient et le ventilateur étant reliés fluidiquement par un tube respiratoire selon l'une quelconque des revendications 1 à 12.
